# EUROPEAN PATENT SPECIFICATION

(11) **EP 3 790 019 B1**
(45) Date of publication and mention of the grant of the patent: **14.08.2024**
(21) Application number: 20194416.2
(22) Date of filing: 03.09.2020
(51) Int. Cl.: G16H 20/40, G16H 50/20, G16H 50/70

(54) **ROBOTICALLY-ASSISTED SURGICAL PROCEDURE FEEDBACK TECHNIQUES BASED ON CARE MANAGEMENT DATA**
ROBOTISCH UNTERSTÜTZTE CHIRURGISCHE PROZEDURRÜCKKOPPLUNGSTECHNIKEN AUF DER BASIS VON PFLEGEVERWALTUNGSDATEN
TECHNIQUES DE RÉTROACTION DE PROCÉDURES CHIRURGICALES ASSISTÉES PAR ROBOT SUR LA BASE DE DONNÉES DE GESTION DE SOINS

(30) Priority: 04.09.2019 US 201916560793
(43) Date of publication of application: 10.03.2021
(73) Proprietor: Medtech SA, 34000 Montpellier (FR)
(72) Inventor: SPOONER, Ted, Grand Rapids, MI 49506 (US); NASH, Jason, Warsaw, IN 46580 (US); COUTURE, Pierre, Montreal, Québec H4C 2J8 (CA); HARTMAN, William, Warsaw, IN 46582 (US)
(74) Representative: Taor, Simon Edward William

(56) References cited:
- WO-A1-2017/214656
- HOSSAIN BELAYAT ET AL: "Implanted Knee Kinematics Prediction: comparative performance analysis of machine learning techniques", 2018 JOINT 7TH INTERNATIONAL CONFERENCE ON INFORMATICS, ELECTRONICS & VISION (ICIEV) AND 2018 2ND INTERNATIONAL CONFERENCE ON IMAGING, VISION & PATTERN RECOGNITION (ICIVPR), IEEE, 25 June 2018 (2018-06-25), pages 544 - 549, XP033518342, DOI: 10.1109/ICIEV.2018.8640999
- RAMKUMAR PREM N ET AL: "Remote Patient Monitoring Using Mobile Health for Total Knee Arthroplasty: Validation of a Wearable and Machine Learning-Based Surveillance Platform", THE JOURNAL OF ARTHROPLASTY, CHURCHILL LIVINGSTONE, AMSTERDAM, NL, vol. 34, no. 10, 16 May 2019 (2019-05-16), pages 2253 - 2259, XP085833390, ISSN: 0883-5403, [retrieved on 20190516], DOI: 10.1016/J.ARTH.2019.05.021

## Description

### BACKGROUND

Diagnostics are used to evaluate a patient to determine whether the patient needs a surgical procedure, such as a total hip arthroplasty, ligament repair, knee replacement, shoulder replacement, or the like. These procedures are performed hundreds of thousands of times a year in the United States. Surgical advancements have allowed surgeons to use preoperative planning, display devices, and imaging, to improve diagnoses and surgical outcomes. Computer-assisted surgery is a growing field that encompasses a wide range of devices, uses, procedures, and computing techniques, such as surgical navigation, pre-operative planning, and various robotic techniques. However, when performing these techniques, patient outcomes are difficult to determine, and sometimes remain unknown. Hossain Beleyat et al: "Implanted knee kinematics prediction: comparative performance analysis of machine learning technologies" discusses Machine Learning methods for predicting total knee arthroplasty outcomes; Ramkumar Prem N et al: "Remote patient monitoring using mobile health for total knee arthroplasty: Validation of a wearable and machine learning-based surveillance platform" discusses remote patient monitoring systems for measuring outcomes following total knee arthroplasty procedures.; WO2017/214656 A1 discusses a computer assistant for a surgeon with a graphical representation of a dynamic knee score for a knee surgery.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates a robotic surgery and feedback system in accordance with some embodiments.
FIGS. 2A-2C illustrate user interfaces for providing feedback in accordance with some embodiments.
FIG. 3 illustrates a machine learning engine for determining feedback in accordance with some embodiments.
FIG. 4 illustrates configurations for generating patient outcome information in accordance with some embodiments.
FIG. 5 illustrates a system for pre-operative or intra-operative surgical procedure feedback in accordance with some embodiments.
FIG. 6 illustrates a flowchart illustrating a technique for providing intra-operative surgical procedure feedback in accordance with some embodiments.
FIG. 7 illustrates a flowchart illustrating a technique for determining a postoperative protocol for a patient using machine learning techniques in accordance with some embodiments.
FIG. 8 illustrates a block diagram of an example machine upon which any one or more of the techniques discussed herein may perform in accordance with some embodiments.

### DETAILED DESCRIPTION

Postoperative recovery may be critical to positive long-term outcomes for orthopedic surgical procedures, but monitoring and controlling postoperative recovery has historically been a major challenge. Monitoring compliance with exercise regiments or limitations has relied primary on voluntary patient feedback, controlled therapy sessions, or periodic office visits. The lack of routine and reliable postoperative monitoring data makes adjusting postoperative recovery protocol recommendations difficult. One aspect of the current disclosure discusses methods and systems for collecting and analyzing real-time or near real-time data indicative of recovery progress, such as protocol compliance, range of motion, patient pain or comfort level, among other parameters. Collection of postoperative patient data allows for development of a database of information that may be correlated to final outcomes, which allows for training of machine-learning algorithms capable of recommending protocols and/or protocol changes based on newly received post-operative patient data. As discussed herein, postoperative patient data may be collected through use of mobile devices, such as smart phones and smart wearables (e.g., smart watch or similar wearable sensor devices). The smart phone may include interactive applications to collect patient engagement and feedback, while the smart wearables may be used to collect objective measurement data (e.g., range of motion, gait, or step count, among others).

Another aspect of improving patient outcomes involves correlating intra-operative information collected during a procedure with final patient outcomes. Collection and correlation of objective intraoperative measures, such as soft tissue tension, implant orientation, etc., with postoperative protocol and outcome data provides another avenue to guide postoperative care towards successful outcomes. For example, in a robotically guided knee or hip arthroplasty, the robotically guided surgical plan may be correlated with postoperative results including postoperative recovery protocols used to obtain the final results. With the objective data obtained from a robotically guided procedure, machine-learning algorithms can be trained to assist in guiding selection of the best postoperative protocols to obtain positive outcomes. Patient mobile devices can be programmed to monitor and guide post-operative recovery protocols automatically.

Intraoperative collection of robotic surgical data may be used, in an example, with a final anatomy state (e.g., a final state of a knee, hip, or shoulder) along with postoperative outcomes in a machine-learning algorithm to develop a postoperative protocol model for generating a postoperative plan. The intraoperative robotic data may be used as training data, labeled with positive or negative patient outcomes based on postoperative steps taken by patients. A postoperative protocol may be generated by the model based on an input of a final patient state or intraoperative patient data.

A postoperative protocol as described herein may include rehabilitation, such as physical therapy or occupational therapy. The postoperative protocol may include education (e.g., recommended reading) for a patient, other types of therapy (e.g., heat or cold therapy), other recommended exercises or routines, or the like. Feedback may be provided during a postoperative protocol (which may optionally be fed back into a machine-learning algorithm to update a model), which may be used to update the postoperative protocol (e.g., via the model). Updates and feedback from a patient may be provided to a surgeon for training.

Systems and methods for determining and providing feedback to a clinician, surgeon, patient, or caretaker related to postoperative care after a surgical procedure based on data collected and analyzed are described herein. The systems and methods herein may provide feedback from a robotic surgical procedure where a robotic surgical device may perform or record an operation or information about a surgical procedure. For example, the robotic surgical device may be used to perform a portion of a surgical procedure, such as a soft tissue balancing test in a knee arthroplasty, and record specific detailed information about the surgical procedure. The information recorded may include parameters of operations performed by the robotic surgical device, patient information, details about the procedure, metadata (e.g., date, time, temperature, pressure, etc.), or the like. For example, in a robotically assisted knee arthroplasty, the robot may assist in operations such as soft tissue balancing and bone resections. Specific objective parameters from these operations may be recorded in association with this patient (e.g., soft tissue tension numbers for medial and lateral sides of the knee and resection angles). Recording of these types of specific information may then be correlated with the specific patient's outcome at a later date when the success of the overall procedure can be fully evaluated. Recording and correlation of pre-operative, intra-operative, and post-operative information may then be utilized in real-time to provide evidence based recommendations for procedures encountering similar surgical situations.

A postoperative protocol may be provided, including for example physical therapy, occupational therapy, stretches or exercises, education, checkup dates or timelines, or the like. Data visualization may be used to present feedback to a clinician regarding a patient's success following the postoperative plan, range of motion information, tracking information related to different aspects of a surgical procedure and resulting patient outcomes, etc.

The systems and methods described herein may be used to provide a recommendation an alert when a critical issue or recommendation event is identified (e.g., a change to a postoperative protocol). The recommendation or alert may be determined based on past recorded information, patient provided outcomes, sensor data (e.g., from a sensor coupled to an implant), metadata, a model trained in a machine learning system, or the like. Information collected during a surgical procedure may be automatically stored at a robotic surgical device. The stored information may be transmitted or transferred to a secure server or database for later use. In an example, the information may be anonymized, such as until a user (e.g., a patient) opts in to using the data. In an example, the information may be stored in a manner that makes the data inaccessible or encrypted (e.g., until the patient opts in). In certain examples, the data is never directly associated with a specific patient, but rather the pre-operative, intra-operative, and post-operative information is anonymously correlated to create patterns of pre-operative deformities correlated with successful intra-operative interventions.

The systems and methods described herein may use received patient opt in, such as by receiving submission of patient outcome information (e.g., pain details, an assessment, range of motion, a patient satisfaction score, such as a forgotten knee score, a Western Ontario and McMaster Universities Arthritis Index (WOMAC) score, shoulder assessment, hip assessment, etc.). After the patient has opted in, the systems and methods described herein may retrieve stored information from a surgical procedure and relate the stored information to outcome information. The determined relationship (including, for example the stored information and the outcome information) may be used to train a model using machine learning. The model may then be used to evaluate a subsequent surgical procedure to determine whether a recommendation or alert may be issued.

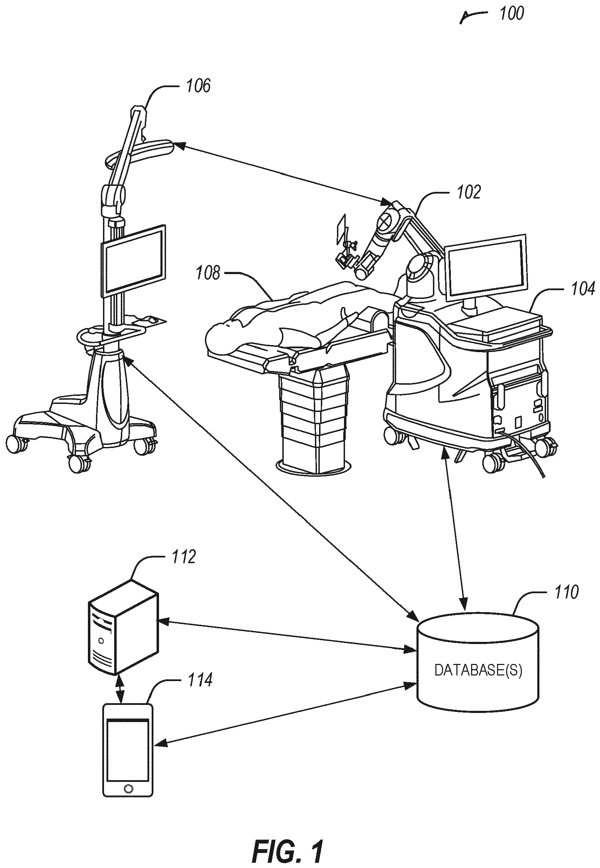

FIG. 1 illustrates a robotic surgery and feedback system 100 in accordance with some embodiments. The system 100 includes a robotic surgical device 102, which may include a computing device 104 (e.g., a processor and memory for performing instructions). The system 100 includes a database 110. The robotic surgical device 102 may be used to perform a portion of a surgical procedure on a patient 108 (e.g., a partial or total knee arthroplasty, a hip arthroplasty, a shoulder arthroplasty, etc.). The robotic surgical device 102 (e.g., via the computing device 104) may store or send data, such as information about an action taken by the robotic surgical device 102 during the portion of the surgical procedure. The data may be sent to the database 110, which may be in communication with a server 112 or user device 114. The system may include a display device 106, which may be used to issue an alert, display information about a recommendation, or receive a request for additional information from a surgeon.

The system 100 may be used to generate or collect data pre-operatively or intra-operatively regarding aspects of a surgical procedure, such as actions taken by the robotic surgical device 102, input from a surgeon, patient anatomy information, or the like. The data may be saved, such as in the database 110, which may be accessed via a server 112 or a user device 114. In an example, the system 100 may generate a code that may be given to a patient after a procedure, such as in a physical copy or electronically sent (e.g., to the user device 114) with the code. The patient may log in to a landing page or portal (e.g., a website), set up an account for the procedure or for the user, and enter the code. The code may be used to access the data from the database 110, where log files of data collected during a procedure may be stored, such as in an anonymized way. Once accessed using the code, the system 100 may retrieve the data for the procedure or the patient. For example, the data may be migrated to the server 112 which may be separate from the database 110 or other server storing the anonymized data. Patient information (e.g., outcome data) may be correlated, combined, or otherwise tied to the procedure or patient data retrieved via the code. The code discussed here is simply one, of potentially many, mechanisms for anonymizing the pre-operative, intra-operative, and post-operative data from the patient information. In this or a similar manner, the system may create a database of deformities, corrective interventions, and outcomes that are correlated, but which are not specifically traceable back to an individual patient without the code or some other identifying information held in a different database. In another example, instead of or in addition to using a code, a machine-readable identifier (e.g., a barcode, a QR code, etc.) may be used. In yet another example, a biometric identification may be used (e.g., fingerprint). Further references to the code throughout this disclosure may include one or more of these identification techniques.

In an example, the patient may be presented one or more questions (e.g., a survey), or asked to supply additional information (e.g., a link to other information, such as a physical or occupational therapy report). In an example, the patient may report outcome information periodically. Any information provided by the patient, after the patient opts in to the system 100 by supplying the code may be stored for processing.

Using collected data, a machine learning model may be trained. For example, pre-, intra-, or post- operative data, and corresponding postoperative outcomes (e.g., range of motion, pain ratings, etc.) may be used as labeled data to train the model. After training, a postoperative protocol may be generated using the model. The postoperative protocol may include exercises, education, timelines, or the like. The postoperative protocol may be generated from the model using the data and outcomes. For example, postoperative steps taken by previous patients and collected data for those patients may be compared to outcomes. When outcomes are generally positive, the postoperative steps may be positively correlated with the collected data. When outcomes are generally negative, the postoperative steps may be negatively correlated with the collected data. In this manner, data for a new patient may be collected, and using the machine learning trained model, a postoperative protocol may be output, the postoperative protocol having steps that are correlated to positive outcomes for data similar to the new patient's data.

In an example, data generated or collected by the surgical robotic device 102 may include data relative to ordinary use of the surgical robotic device 102, data collected on robot use during a procedure, data on use of aspects of the system 100 such as, time spent by a user on a user interface, number of clicks or key presses on a user interface, an adjustment or change to a plan (e.g., pre-operative plan), differences between an original plan and a final plan, duration of use of the surgical robotic device 102, software stability or bugs, or the like. The data collected may be linked using a clinical mechanism to determine whether outcomes are improved. In an example, the data collected may be surgeon specific.

Pre-operative data may include medical imaging of a target procedure site, statistical representations of bones involved in the procedure, virtual models generated of the target procedure site (e.g., a three-dimensional model of a knee joint (distal femur and proximal tibia)), planned implant position and orientation on the virtual models, and planned resections or similar operations to be performed, among other things. Intra-operative data may include soft tissue tension measurements of a joint, intra-operative adjustments to pre-operative plan (implant position/orientation and resections), actual resection parameters (e.g., position and orientation of resections on distal femur) and final implant location (in reference to known landmarks and/or pre-operative plan). Finally, post-operative data may include objective data obtained from follow up medical imaging or other mechanisms to assess implant performance or procedural success, but may also focus on subjective patient impressions and physical performance (e.g., range of motion and strength).

During a procedure, such as in the operating room, a data analytic program may be run (e.g., on the surgical robotic device 102), for example in the background of a knee application. The program may run a simulation in real time to provide insight to the user (e.g., by providing a recommendation, confirmation, or alert). The insight may be based on statistical analysis of historical data and intra-operative decisions or actions taken by the surgeon or using the surgical robotic device 102. In an example, at any step of a given procedure, a recommendation, confirmation, or alert to the surgeon may be updated based on latest actions. This may result in better patient outcome. The data generated or stored by the surgical robotic device 102 during the procedure may be stored (e.g., on the database 110) and used in future procedures.

Postoperative data collection may include patient-submitted data (e.g., a pain rating, compliance, exercises performed, comfort level, perceived range of motion, etc.), clinician-submitted data (e.g., based on range of motion tests performed with clinician supervision, evaluations by a clinician, or the like), or objective data (e.g., tested range of motion data, number of visits to a physical therapist, compliance with protocol, or the like).

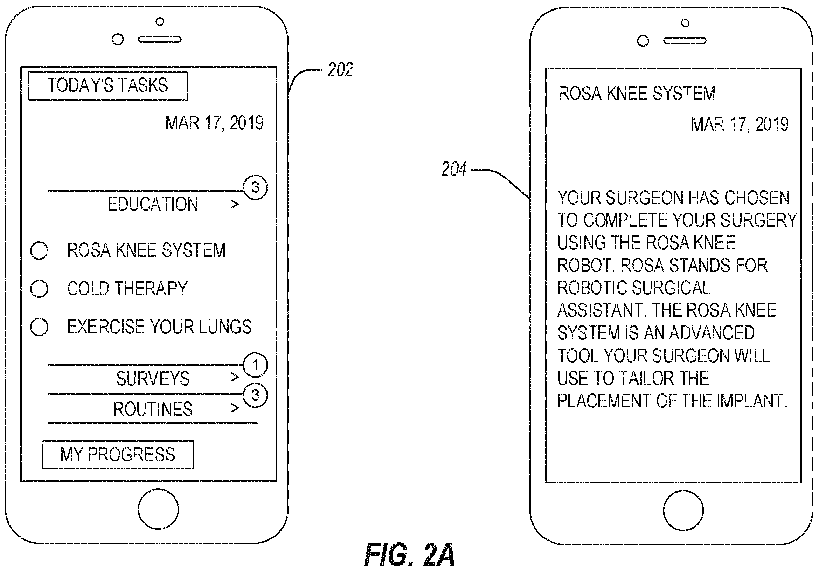

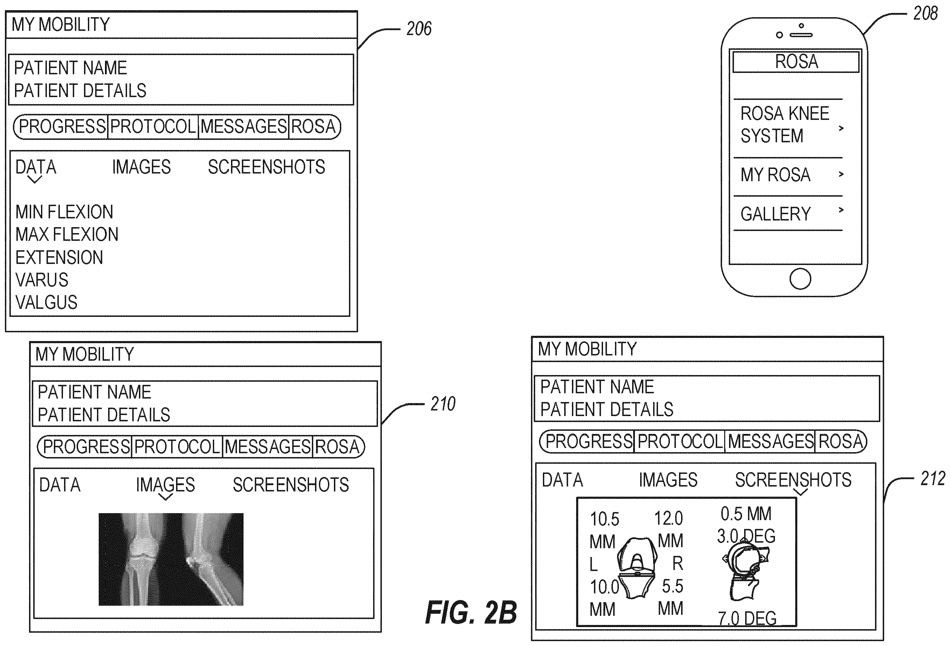

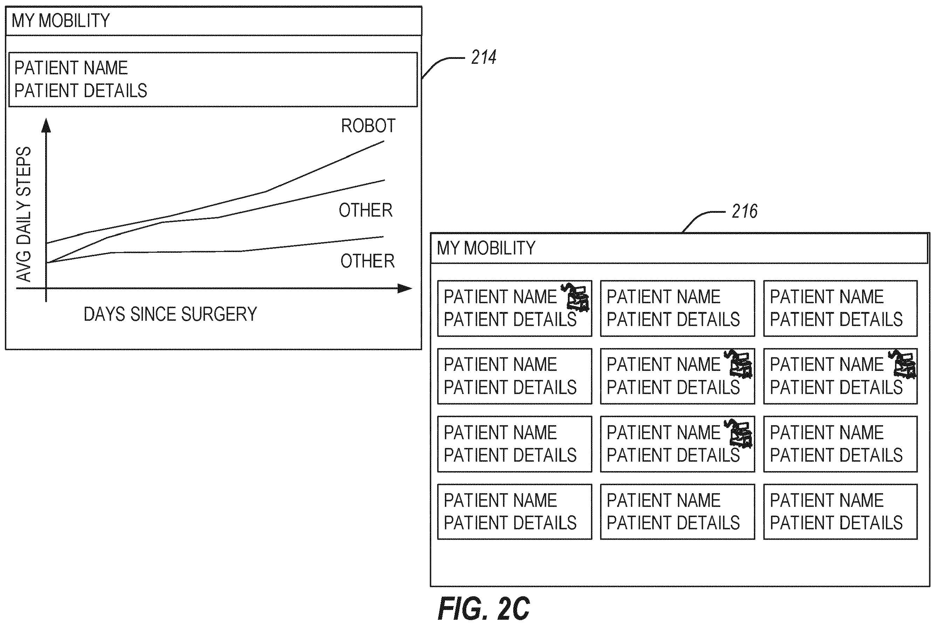

FIGS. 2A-2C illustrate user interfaces for providing feedback in accordance with some embodiments. FIG. 2A illustrates example patient user interfaces 202 and 204. FIG. 2B illustrates example surgeon or clinician user interfaces 206, 208, 210, and 212. FIG. 2C illustrates example user interfaces 214 and 216 for data visualization and analysis for one or a plurality of patients. These example user interfaces may be displayed on a computer display (e.g., a laptop or a separate display connected to a desktop computer), a mobile device (e.g., a phone), a tablet, or the like.

In FIG. 2A, patient user interface (UI) 202 includes portions of a postoperative protocol for a patient. For example, education, surveys, or routines may be shown to a patient as steps in a postoperative protocol. Tasks may be shown daily, weekly, monthly, or the like. Progress may be displayed to show a patient changes over time. Patient UI 204 illustrates an example of educational material provided to the patient, for example when the patient selects the education task on UI 202. The patient may be educated on robotic surgery, which may be useful for a patient to explain their surgery to friends and family. This may provide a positive experience for the patient, which will make them more comfortable with the procedure. This also allows a patient to become comfortable with using their devices (e.g., phone or smartwatch) to educate themselves in a low stress way before surgery. Then after surgery, the patient may be familiar with using the devices for education, which may make them more likely to use the devices for education postoperatively.

FIG. 2B includes various example UIs including a patient history UI 206, example education for showing to a patient on a UI 208, imaging information on UI 210, and technical details on UI 212.

FIG. 2C includes data visualization on UI 214, which may for example, illustrate relative achievements, such as quicker recovery or greater mobility/range of motion with a robotic surgical device compared to a surgery without a robotic surgical device. The data visualization UI 214 may be based on all or a subset of patients of a clinician (e.g., a surgeon), a practice (e.g., a group of surgeons), a hospital, a region, or the like. UI 216 illustrates a quick view of patient information for a plurality of patients (e.g., postoperative patients for a surgeon or a physical therapist, such as a set of patients scheduled for an appointment in a particular day or week). In an example, UI 216 may not include patient identifiable information. UI 216 may show patient outcome data for patients similar to a current patient (e.g., similar comorbidities).

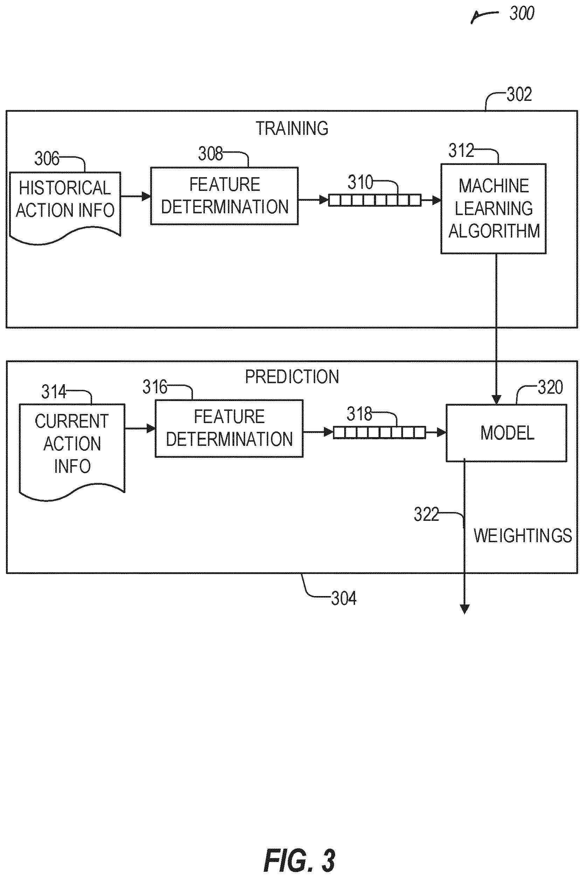

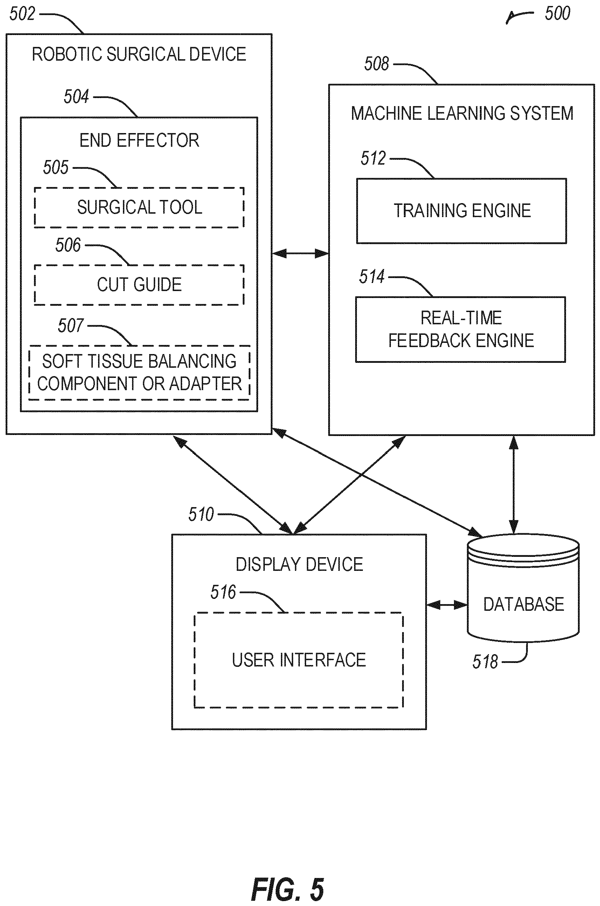

FIG. 3 illustrates a machine learning engine for determining feedback in accordance with some embodiments. A system may calculate one or more weightings for criteria based upon one or more machine learning algorithms. FIG. 3 shows an example machine learning engine 300 according to some examples of the present disclosure. Machine learning engine 300 may be part of the system 100 of FIG. 1, for example implemented using the database 110, the server 112, etc., or the machine learning system 508 of FIG. 5, described below.

Machine learning engine 300 utilizes a training engine 302 and a prediction engine 304. Training engine 302 inputs historical information 306 for historical actions of a robotic surgical device, or stored or generated at a robotic surgical device, for example, into feature determination engine 308. Other historical information 306 may include preoperative data (e.g., comorbidities, varus/valgus data, pain, range of motion, or the like), intraoperative data (e.g., implant used, procedure performed, etc.), or postoperative data (e.g., range of motion, final state of patient anatomy, postoperative steps taken, such as physical therapy, education, etc., pain data, or the like). The historical action information 306 may be labeled with an indication, such as a degree of success of an outcome of a surgical procedure, which may include pain information, patient feedback, implant success, ambulatory information, or the like. In some examples, an outcome may be subjectively assigned to historical data, but in other examples, one or more labelling criteria may be utilized that may focus on objective outcome metrics (e.g., range of motion, pain rating, survey score, a patient satisfaction score, such as a forgotten knee score, a WOMAC score, shoulder assessment, hip assessment, or the like).

Feature determination engine 308 determines one or more features 310 from this historical information 306. Stated generally, features 310 are a set of the information input and is information determined to be predictive of a particular outcome. Example features are given above. In some examples, the features 310 may be all the historical activity data, but in other examples, the features 310 may be a subset of the historical activity data. The machine learning algorithm 312 produces a model 320 based upon the features 310 and the labels.

In the prediction engine 304, current action information 314 (e.g., preoperative data, a final state of patient anatomy, such as a final knee state, a surgical plan, an action to be taken or a last action taken, such as by a robotic surgical device, or the like) may be input to the feature determination engine 316. Feature determination engine 316 may determine the same set of features or a different set of features from the current information 314 as feature determination engine 308 determined from historical information 306. In some examples, feature determination engine 316 and 308 are the same engine. Feature determination engine 316 produces feature vector 318, which is input into the model 320 to generate one or more criteria weightings 322. The training engine 302 may operate in an offline manner to train the model 320. The prediction engine 304, however, may be designed to operate in an online manner. It should be noted that the model 320 may be periodically updated via additional training or user feedback (e.g., an update to a technique or procedure).

The machine learning algorithm 312 may be selected from among many different potential supervised or unsupervised machine learning algorithms. Examples of supervised learning algorithms include artificial neural networks, Bayesian networks, instance-based learning, support vector machines, decision trees (e.g., Iterative Dichotomiser 3, C4.5, Classification and Regression Tree (CART), Chi-squared Automatic Interaction Detector (CHAID), and the like), random forests, linear classifiers, quadratic classifiers, k-nearest neighbor, linear regression, logistic regression, and hidden Markov models. Examples of unsupervised learning algorithms include expectation-maximization algorithms, vector quantization, and information bottleneck method. Unsupervised models may not have a training engine 302. In an example embodiment, a regression model is used and the model 320 is a vector of coefficients corresponding to a learned importance for each of the features in the vector of features 310, 318.

Once trained, the model 320 may output a postoperative protocol for a patient based on a final state of patient anatomy, or pre- or intra-operative data. In another example, the model 320 may predict a postoperative protocol for a patient pre- or intra-operatively based on available data.

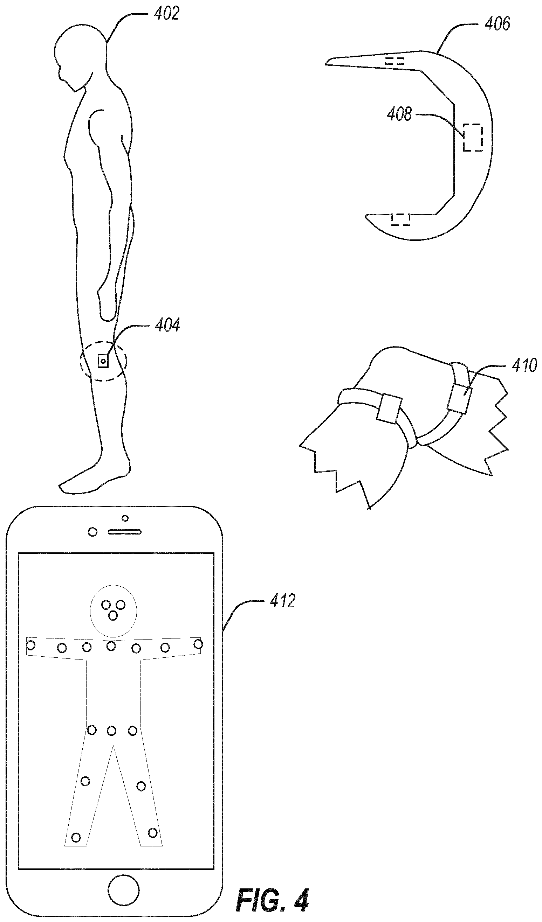

FIG. 4 illustrates configurations for generating objective patient outcome information in accordance with some embodiments. A sensor may be included in an implantable orthopedic device. The implant may act as a host for the sensor or be the sensor itself. FIG. 4 illustrates an example sensor 404 placements in or on a knee of a patient 402 in accordance with some examples. The sensor 404 may be placed at various locations on an implant, on a bone or attached to the knee outside the skin. The placement of sensor 404 may vary according to the type of implant, the properties of the bone, or the type of sensor. The sensor 404 may be used to measure or track patient movement, range of motion, pain, fit, a patient satisfaction score, such as a forgotten knee score, a WOMAC score, or the like. The information measured or tracked by the sensor 404 may be saved at a component of the sensor 404 or may be transmitted wirelessly (e.g., using near field communication, RFID, other wireless protocols, such as Bluetooth or Wi-Fi, or the like), such as to a computer or wireless device (e.g., a mobile phone, tablet, wearable device, or the like). In another example, the sensor may be located within a shoulder of a patient (e.g., within an implant).

FIG. 4 depicts various placements on an implant 406 for a sensor 408. The examples of FIG. 4 may include an implanted sensor 408 (e.g., a first sensor, a post-operative sensor) associated with a knee joint of the patient. The sensors depicted in FIG. 4 are merely illustrative and other sensors in other locations may be used in examples according to this disclosure.

In an example, a wearable sensor device 410 may be used in addition to or instead of the sensor 404 or 408. In an example, a wearable sensor device may be an off-the-shelf consumer wearable device such as, for example, Fitbit, Jawbone, Apple Watch, or other consumer wearable electronic devices, or sensor device may be a custom sensor that is configured to be worn by a patient to collect pre-operative data or post-operative data. Implanted sensors may be employed to collect pre-operative or post-operative data. In some cases, the sensor may be attached to the patient on, proximate or near the site where an orthopedic surgery may be performed. The sensor 410 may be attached via a garment or strap, however it may also be attached to the patient, for example, via a temporary adhesive.

In some examples, knee sensor technology may include a sensor or sensors to monitor steps, forces, friction, temperature, or the like. Sensors may provide useful data from positions throughout the body. In other examples, shoulder sensor technology may include a sensor or sensors to monitor movement, such as rotation of the shoulder, forces, friction, temperature, or the like.

The example sensors 404 or 408 or 410 may include one or more of an accelerometer, a temperature sensor, a force sensor, a resistance sensor, a tachometer, a healing indicator, a pH measure sensor, a tension or compression sensor, callous formation sensing tape, a strain sensor (e.g., strain gauge), a gyroscope or the like. The example sensors 404 or 408 or 410 may include active sensors and inactive sensors.

Sensor data may be collected data constantly, or periodically. The collected data may be transmitted, such as routinely, occasionally, or in response to an activation. Activation of a sensor may be based on patient permission, such as post-operation permission when a sensor is included in an implant without pre-operation patient permission to activate. In some examples, access to a sensor in an implant may be an encrypted permission and may rely on an activation code. The data from the sensor may be used to compare a pre-operative plan or an intra-operatively changed plan, to final implant parameters.

In another example, instead of using sensors affixed to a patient via straps or implants, a mobile device with a camera, such as a phone, may be used to capture a user performing an exercise or moving.

FIG. 5 illustrates a system 500 for pre-operative or intra-operative surgical procedure feedback in accordance with some embodiments. The system 500 includes a robotic surgical device 502, which may include an end effector 504, such as to attach or manipulate a surgical tool 505, a cut guide 506, a soft tissue balancing component or adapter 507, or the like. The robotic surgical device 502 may output data to a machine learning system 508, a display device 510, or a database 518. In an example, the machine learning system 508 may output information to the display device 510 or a database 518. The display device may retrieve information stored in the database 518. The display device 510 may be used to display a user interface 516. In an example, the machine learning system 508 includes a training engine 512 and a real-time feedback engine 514.

The robotic surgical device 502 may be used to perform a portion of a surgical procedure on a patient. A processor may be coupled to memory (e.g., on the robotic surgical device 502 or the machine learning system 508). The processor may be used to record an action taken by the robotic surgical device 502, such as during the portion of the surgical procedure. The processor may query a database to retrieve information about related prior surgical procedures. In an example, the information may include at least one result or next action taken after the action (e.g., a recommendation or an alert). The processor may determine a recommended change, such as based on the information, to the portion of the surgical procedure or a future aspect of the surgical procedure. The recommended change may be a change as performed by the robotic surgical device 502. The processor may output the recommendation (e.g., to the display device 510). The output may include using the processor or the display device 510 to intraoperatively provide the recommendation to a surgeon operating the robotic surgical device 502. The output may be performed without surgeon input as an alert, or in response to receiving a request for the recommendation, such as on the user interface 516.

In an example, the machine learning system 508 may train using the related prior surgical procedures, including, for example, at least one action taken by the robotic surgical device 502 or at least one corresponding outcome. The at least one corresponding outcome may be based on a patient outcome received from the patient. In an example, the processor may submit a plan to the machine learning system 508 to receive feedback preoperatively, intraoperatively, or postoperatively. In an example, the machine learning system 508 may simulate the portion of the surgical procedure to determine one or more postoperative protocols. The machine learning system 508 may select the recommended change from the plurality of recommended changes, such as based on outcome likelihoods of the one or more postoperative protocols.

In an example, the information about related prior surgical procedures may include patient-specific information about a past procedure performed on the patient (e.g., during a revision surgery, information regarding the previous primary surgery may be considered). In another example, the information about related prior surgical procedures includes demographic-specific information or comorbidity information corresponding to the patient. For example, the demographic-specific information may include at least one of patient size (e.g., height, weight, gender, which knee, hip, or shoulder, etc.), surgical procedure type, patient age, or the like.

In an example, the processor may store anonymized data related to the action taken by the robotic surgical device on a first server, receive a code entered by the patient, and pull the anonymous data onto a second server. The processor may tie patient identifying information to the anonymized data on the second server.

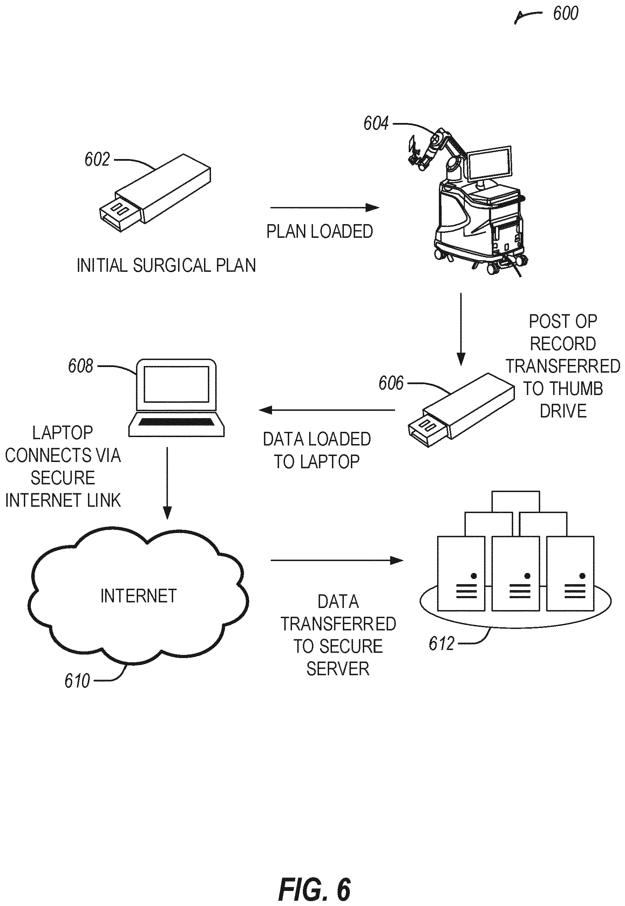

FIG. 6 illustrates a data flow diagram 600 for storing actions of a robotic surgical device in accordance with some embodiments. The diagram 600 includes a plurality of operations for loading, saving, and transmitting surgical information among different devices. In an example, a surgical plan may be generated and saved a thumb drive or other storage device at 602. The initial surgical plan may be delivered to a robotic surgical device via the encrypted thumb drive at 604. During a procedure or operation, data may be collected on the robotic surgical device and may be stored to await transfer. After the operation or procedure (e.g., post-op), the data may be transferred from the robotic surgical device to a server via encrypted thumb drive at 606, loaded to an internet connected computer or device at 608, and uploaded via a secure internet or network link to a server at 610. In an example, the data may be stored per procedure, anonymously on the server. The data may be transferred to the secure server at 612. In an example, a coded patient consent field may be stored as part of the record. The coded patient consent field may include a key or code, which may be decrypted in response to receiving a code or key from the patient.

The diagram 600 illustrates actions which may provide feedback to improve patient outcomes and protect patient privacy. For example, the processes described in FIG. 6 may be opt-in based on patient consent. In an example, some information may be recorded via the robotic surgical device 604 and some information submitted later by a patient. These two types of data may be linked to determine whether any actions taken during a procedure affect the outcome. In an example, patient information or feedback may be sought out at intervals. After some data has been collected, the results may be grouped into positive outcomes or negative outcomes, and trends for actions taken during a procedure that lead to those outcomes may be determined. Outcome or action information may be grouped based on one or more attributes of a procedure, such as patient, surgeon, demographic information about the patient, geographic location, duration of procedure, time of day of procedure, day of week of procedure, date of procedure, number of changes to a plan, or the like. Additional information may be used, such as post-operative actions taken by the surgeon or patient (e.g., surgeon follow-up, education, patient physical therapy, adherence to post-op plan, etc.).

After an outcome is associated with actions taken during a procedure, the actions may be evaluated to determine whether one may have caused the outcome. For example, a plurality of positive outcomes or a plurality of negative outcomes may be compared to determine common actions, or actions that are not shared between positive and negative outcomes. For example, soft tissue balancing information may be evaluated such that positive outcomes may be associated with balanced soft tissue. In an example, patients that do not have soft tissue cut during a knee arthroplasty (e.g., ligament releases) may have better outcomes (e.g., for pain, WOMAC score, forgotten knee, etc.) than patients that have soft tissue cut during a knee arthroplasty.

In an example, using the techniques and systems described herein may benefit patients, surgeons, or the medical community. For example, the techniques and systems described herein may allow for expanded clinical research capabilities, and provide ease of data entry for clinicians because the robotic surgical device may capture data, using a specified format of data collection for efficient analysis, or the like.

At the end of a surgical procedure, a user operating the robotic surgical device may print or electronically transmit a patient record to give to the patient during or after discharge. The patient record may include general implant information, brand, size, surgeon name, date, or the like. The patient record may include a unique code for accessing the information or opting in to further analysis. The patient may, upon initial home recovery, log on to a user interface that describes the need for clinical information, cadence of outcome forms, description of types of questions, etc., and the patient may then "sign up" for a clinical event.

By signing up, the patient may consent to analysis and access of the patient data to create a link between the anonymous record, and future patient report outcomes, and link the two records. The unique code may trigger population of a second data set, which may include a duplication of only the records that patients have consented to be part of the clinical event. Access may be limited except for clinical research teams, or other groups (e.g., the surgeon) based on patient preference. In an example, the patient may fill out a clinical form at 6 months, 1 year, 2 years, etc.

At given relevant review periods, the data is analyzed for changes in satisfaction levels. Statistical analysis may dictate relevance of clinical findings. When a subset of data reveals higher satisfaction for example, regression analysis may be performed over the second data set to determine whether there is a common data point that explains the improved satisfaction (e.g., a specific action or inaction, such as cutting the soft tissue in a knee arthroplasty, or aligning the implant according to a kinematic vs. mechanical approach). For example, a data set is analyzed, a particular subset of patients have higher satisfaction, regression analysis performed, results in findings, such as patients found to have greater femoral rotation, no medial ligament release, or less tibia rotation. This information may be fed back into a machine learning algorithm, such as described herein.

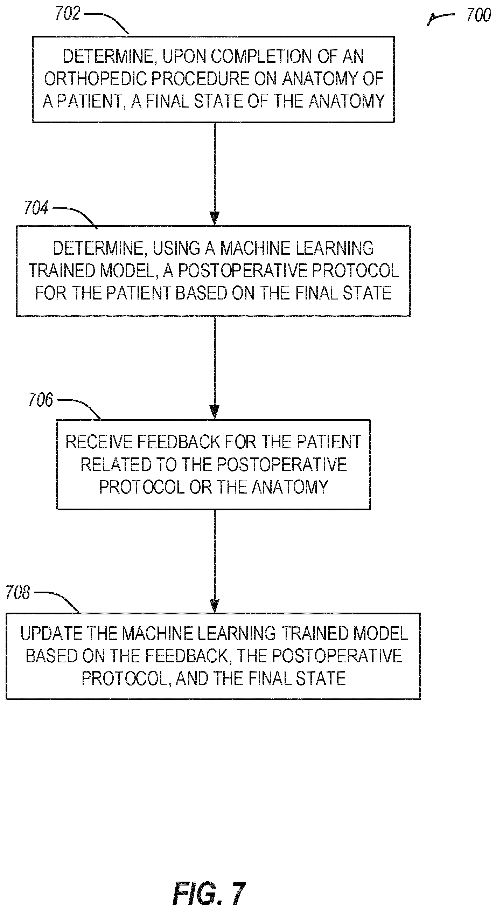

FIG. 7 illustrates a flowchart illustrating a technique 700 for determining a postoperative protocol for a patient using machine learning techniques in accordance with some embodiments. The technique 700 includes an operation 702 to determine, upon completion of an orthopedic procedure on anatomy of a patient, a final state of the anatomy. The final state of the anatomy may include any information about a patient's anatomy at the end of a surgical procedure. For example, the final state may include a final range of motion, a final valus/vargus angle or value for a knee, a final ligament tension or gap distance, a final implant type or size, a or other final information, about a state of the anatomy. The final state may be determined by a surgeon at the end of the procedure, including subjective assessment information or objective evaluation information. One specific example of the final state may include a final knee state. In an example, intraoperative data from a surgical robot may be used to determine the final state.

The technique 700 includes an operation 704 to determine, using a machine learning trained model, a postoperative protocol for the patient based on the final state. In an example, the machine learning trained model is trained using preoperative information about a patient.

The technique 700 includes an operation 706 to receive feedback for the patient related to the postoperative protocol or the anatomy. In an example, the feedback includes range of motion information and pain information. In an example, the pain information may be detected using motion capture of the user, based on identifying that the user is compensating when moving.

The technique 700 includes an operation 708 to update the machine learning trained model based on the feedback, the postoperative protocol, and the final state. The anatomy may be, for example a knee of the patient and the final state may include a final knee state, for example based on five variables.

The technique 700 may include receiving intraoperative information, for example from a robotic surgical device, during the orthopedic procedure, and wherein updating the machine learning trained model includes using the intraoperative information.

The technique 700 may include receiving, intraoperatively, a predicted final state of the anatomy. The prediction may be input to the machine learning trained model to predict a postoperative protocol for the patient based on the predicted final state. The predicted postoperative protocol may be output (e.g., intraoperatively) for display.

The technique 700 may include determining a postoperative trajectory for the patient based on the feedback, the postoperative protocol, and the final state. This may include generating, using the machine learning trained model, a change to the postoperative protocol based on the postoperative trajectory. A postoperative trajectory may include an identification of whether a patient is on track with completing tasks of the postoperative protocol. The trajectory may include or be based on a number of tasks completed, a percentage of tasks completed, a number of tasks failed, a number of pain rating, range of motion information, or trends of one or more of these values over time. The trajectory may be determined from periodic updates from the patient, clinicians (e.g., a physical therapist, a surgeon, etc.), or sensor data (e.g., daily steps monitored by an app or phone, movement of a shoulder tracked using a wrist-based device, such as a watch, etc.). The updates or feedback may be fed as inputs into a machine learning model, with an output including whether a change in postoperative protocol may result in a more successful outcome for a patient (more successful meaning, for example less pain over time, improved range of motion, etc.).

The technique 700 may include receiving motion of the patient captured by a camera of a mobile device to determine the feedback. The technique 700 may include recording an action taken by a robotic surgical device during a portion of the orthopedic procedure, determining a recommendation, using the machine learning trained model, to the portion of the surgical procedure performed by the robotic surgical device. The recommendation may be output by, for example, intra-operatively providing the recommendation to a surgeon operating the robotic surgical device.

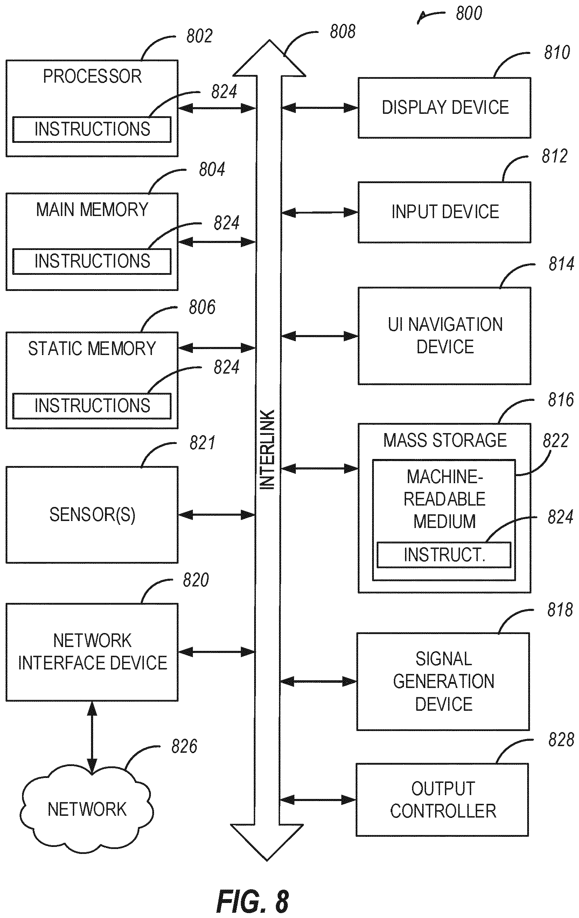

FIG. 8 illustrates a block diagram of an example machine 800 upon which any one or more of the techniques discussed herein may perform in accordance with some embodiments. In alternative embodiments, the machine 800 may operate as a standalone device or may be connected (e.g., networked) to other machines. In a networked deployment, the machine 800 may operate in the capacity of a server machine, a client machine, or both in server-client network environments. In an example, the machine 800 may act as a peer machine in peer-to-peer (P2P) (or other distributed) network environment. The machine 800 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Machine (e.g., computer system) 800 may include a hardware processor 802 (e.g., a central processing unit (CPU), a graphics processing unit (GPU), a hardware processor core, or any combination thereof), a main memory 804 and a static memory 806, some or all of which may communicate with each other via an interlink (e.g., bus) 808. The machine 800 may further include a display unit 810, an alphanumeric input device 812 (e.g., a keyboard), and a user interface (UI) navigation device 814 (e.g., a mouse). In an example, the display unit 810, input device 812 and UI navigation device 814 may be a touch screen display. The machine 800 may additionally include a storage device (e.g., drive unit) 816, a signal generation device 818 (e.g., a speaker), a network interface device 820, and one or more sensors 821, such as a global positioning system (GPS) sensor, compass, accelerometer, or other sensor. The machine 800 may include an output controller 828, such as a serial (e.g., Universal Serial Bus (USB), parallel, or other wired or wireless (e.g., infrared (IR), near field communication (NFC), etc.) connection to communicate or control one or more peripheral devices (e.g., a printer, card reader, etc.).

The storage device 816 may include a machine readable medium 822 on which is stored one or more sets of data structures or instructions 824 (e.g., software) embodying or utilized by any one or more of the techniques or functions described herein. The instructions 824 may also reside, completely or at least partially, within the main memory 804, within static memory 806, or within the hardware processor 802 during execution thereof by the machine 800. In an example, one or any combination of the hardware processor 802, the main memory 804, the static memory 806, or the storage device 816 may constitute machine readable media.

While the machine readable medium 822 is illustrated as a single medium, the term "machine readable medium" may include a single medium or multiple media (e.g., a centralized or distributed database, and/or associated caches and servers) configured to store the one or more instructions 824. The term "machine readable medium" may include any medium that is capable of storing, encoding, or carrying instructions for execution by the machine 800 and that cause the machine 800 to perform any one or more of the techniques of the present disclosure, or that is capable of storing, encoding or carrying data structures used by or associated with such instructions. Non-limiting machine-readable medium examples may include solid-state memories, and optical and magnetic media.

The instructions 824 may further be transmitted or received over a communications network 826 using a transmission medium via the network interface device 820 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, the network interface device 820 may include one or more physical jacks (e.g., Ethernet, coaxial, or phone jacks) or one or more antennas to connect to the communications network 826. In an example, the network interface device 820 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 800, and includes digital or analog communications signals or other intangible medium to facilitate communication of such software.

Method examples described herein may be machine or computer-implemented at least in part. Some examples may include a computer-readable medium or machine-readable medium encoded with instructions operable to configure an electronic device to perform methods as described in the above examples. An implementation of such methods may include code, such as microcode, assembly language code, a higher-level language code, or the like. Such code may include computer readable instructions for performing various methods. The code may form portions of computer program products. Further, in an example, the code may be tangibly stored on one or more volatile, non-transitory, or non-volatile tangible computer-readable media, such as during execution or at other times. Examples of these tangible computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

## Claims

1. A computer-implemented method comprising:
determining, upon completion of an orthopedic procedure on anatomy of a patient, a final state of the anatomy, the final state of the anatomy including information describing the anatomy of the patient following the completion of the orthopedic procedure;
determining, using a machine learning trained model, a postoperative recovery protocol for the patient based on the final state, the postoperative patient treatment protocol determined by the machine learning trained model to correlate to positive outcomes for data similar to the patient;
receiving postoperative feedback data from a device associated with the patient, the postoperative feedback data indicative of a postoperative objective outcome metric related to the postoperative recovery protocol or the anatomy;
generating a changed postoperative patient treatment protocol based on updating the machine learning trained model using training data based on the postoperative feedback data, the postoperative recovery protocol, and the final state; and
outputting a patient treatment recommendation, the patient treatment recommendation based on the changed postoperative patient recovery protocol.

2. The method of claim 1, further comprising receiving intraoperative information, from a robotic surgical device, previously gathered during the orthopedic procedure, and wherein updating the machine learning trained model includes using the intraoperative information as updated training data.

3. The method of claim 1, further comprising:
receiving, intraoperatively during a subsequent procedure, a predicted final state of the anatomy;
using the machine learning trained model to predict a postoperative recovery protocol for the patient based on the predicted final state; and
outputting, intraoperatively, the predicted postoperative recovery protocol for display.

4. The method of claim 1, further comprising:
recording an action taken by a robotic surgical device (102) during a portion of the subsequent procedure;
determining a robotic surgical procedure recommendation, using the machine learning trained model, the robotic surgical procedure recommendation including a modification to the portion of the subsequent procedure performed by the robotic surgical device (102); and
outputting the robotic surgical procedure recommendation by intra-operatively providing the robotic surgical procedure recommendation to a surgeon operating the robotic surgical device (102).

5. The method of claim 1, wherein the anatomy is a knee of the patient and the final state includes a final knee state based on at least one of a final range of motion, a final valus/vargus angle, a final ligament tension, a final gap distance, a final implant type, and a final implant size.

6. The method of claim 1, further comprising determining a postoperative patient recovery trajectory for the patient based on the feedback, the postoperative recovery protocol, and the final state; and generating, using the feedback, the postoperative patient treatment protocol, and the final state as inputs to the machine learning trained model, a change to the postoperative recovery protocol based on the postoperative patient recovery trajectory.

7. The method of claim 1, wherein the postoperative feedback data includes range of motion information and pain information is generated based on a patient input to a mobile electronic device; and
the postoperative feedback data is received from the mobile electronic device.

8. The method of claim 1, further comprising determining the feedback based on receiving motion sensor data from a sensor associated with movement of the patient captured by a camera of a mobile device.

9. At least one machine-readable medium including instructions for operation of a computing system, which when executed by a machine, cause the machine to perform operations of any of the methods of claims 1-8.

10. An apparatus comprising means adapted for performing any of the methods of claims 1-8.

11. A system comprising:
a processor; and
memory including instructions, which when executed by the processor, cause the processor to perform operations to:
determine, upon completion of an orthopedic procedure on anatomy of a patient, a final state of the anatomy following the completion of the orthopedic procedure;
determine, using a machine learning trained model, a postoperative recovery protocol for the patient based on the final state;
wherein the machine learning trained model is trained using postoperative recovery protocols, final states, and postoperative feedback data for patients, the postoperative feedback data indicative of a postoperative objective outcome metric related to the postoperative patient treatment protocol or the anatomy; and
a display device configured to present a user interface to identify the postoperative recovery protocol for the patient.

12. The system of claim 11, wherein the instructions further cause the processor to receive intraoperative information, from a robotic surgical device, during the orthopedic procedure, and using the intraoperative information to generate the postoperative recovery protocol using the machine learning trained model.

13. The system of claim 11, wherein the instructions further cause the processor to:
receive, intraoperatively, a predicted final state of the anatomy; and
use the machine learning trained model to predict a postoperative recovery protocol for the patient based on the predicted final state;
wherein the display device is further configured to display the predicted postoperative recovery protocol on the user interface.

14. The system of claim 11, wherein the machine learning trained model is further trained using preoperative information about the patient as input to the machine learning trained model during training, the preoperative information indicative of the postoperative objective outcome metric related to the postoperative patient treatment protocol or the anatomy.

15. The system of any of claims 11-14, wherein the postoperative feedback data includes range of motion information and pain information is generated based on a patient input to a mobile electronic device, and
the postoperative feedback data is received from the mobile electronic device.

## Patentansprüche

1. Computerimplementiertes Verfahren, umfassend:
nach Abschluss einer orthopädischen Prozedur an Anatomie eines Patienten Bestimmen eines endgültigen Zustands der Anatomie, wobei der endgültige Zustand der Anatomie Informationen einschließt, die die Anatomie des Patienten im Anschluss an den Abschluss der orthopädischen Prozedur beschreiben;
Bestimmen eines postoperativen Genesungsprotokolls für den Patienten auf der Basis des endgültigen Zustands unter Verwendung eines mit maschinellem Lernen trainierten Modells, wobei das postoperative Patientenbehandlungsprotokoll durch das mit maschinellem Lernen trainierte Modell bestimmt wird, um positive Ergebnisse für Daten zu korrelieren, die denen des Patienten ähnlich sind;
Empfangen von postoperativen Rückkopplungsdaten von einer dem Patienten zugeordneten Vorrichtung, wobei die postoperativen Rückkopplungsdaten auf eine postoperative objektive Ergebnismetrik in Bezug auf das postoperative Genesungsprotokoll oder die Anatomie hinweisen;
Erzeugen eines geänderten postoperativen Patientenbehandlungsprotokolls auf der Basis von Aktualisieren des mit maschinellem Lernen trainierten Modells unter Verwendung von Trainingsdaten auf der Basis der postoperativen Rückkopplungsdaten, des postoperativen Genesungsprotokolls und des endgültigen Zustands; und
Ausgeben einer Patientenbehandlungsempfehlung, wobei die Patientenbehandlungsempfehlung auf dem geänderten postoperativen Patientengenesungsprotokoll basiert.

2. Verfahren nach Anspruch 1, ferner umfassend Empfangen von intraoperativen Informationen von einer robotischen chirurgischen Vorrichtung, die zuvor während der orthopädischen Prozedur erhoben wurden, und wobei das Aktualisieren des mit maschinellem Lernen trainierten Modells Verwenden der intraoperativen Informationen als aktualisierte Trainingsdaten einschließt.

3. Verfahren nach Anspruch 1, ferner umfassend:
Empfangen eines vorhergesagten endgültigen Zustands der Anatomie intraoperativ während einer anschließenden Prozedur;
Verwenden des mit maschinellem Lernen trainierten Modells, um ein postoperatives Genesungsprotokoll für den Patienten auf der Basis des vorhergesagten endgültigen Zustands vorherzusagen; und
Ausgeben des vorhergesagten postoperativen Genesungsprotokolls intraoperativ zur Anzeige.

4. Verfahren nach Anspruch 1, ferner umfassend:
Aufzeichnen einer durch eine robotische chirurgische Vorrichtung (102) während eines Abschnitts der anschließenden Prozedur vorgenommenen Aktion;
Bestimmen einer Empfehlung für eine robotische chirurgische Prozedur unter Verwendung des mit maschinellem Lernen trainierten Modells, wobei die Empfehlung für eine robotische chirurgische Prozedur eine Modifikation des Abschnitts der anschließenden Prozedur einschließt, der durch die robotische chirurgische Vorrichtung (102) durchgeführt wird; und
Ausgeben der Empfehlung für eine robotische chirurgische Prozedur durch intraoperatives Bereitstellen der Empfehlung für eine robotische chirurgische Prozedur an einen Chirurgen, der die robotische chirurgische Vorrichtung (102) betreibt.

5. Verfahren nach Anspruch 1, wobei die Anatomie ein Knie des Patienten ist und der endgültige Zustand einen endgültigen Kniezustand auf der Basis von mindestens einem von einem endgültigen Bewegungsbereich, einem endgültigen Valus-/Varguswinkel, einer endgültigen Bänderspannung, einem endgültigen Spaltabstand, einem endgültigen Implantattyp und einer endgültigen Implantatgröße einschließt.

6. Verfahren nach Anspruch 1, ferner umfassend Bestimmen einer postoperativen Patientengenesungstrajektorie für den Patienten auf der Basis der Rückkopplung, des postoperativen Genesungsprotokolls und des endgültigen Zustands; und Erzeugen einer Änderung des postoperativen Genesungsprotokolls auf der Basis der postoperativen Patientengenesungstrajektorie unter Verwendung der Rückkopplung, des postoperativen Patientenbehandlungsprotokolls und des endgültigen Zustands als Eingaben in das mit maschinellem Lernen trainierte Modell.

7. Verfahren nach Anspruch 1, wobei die postoperativen Rückkopplungsdaten Bewegungsbereichsinformationen einschließen und Schmerzinformationen auf der Basis einer Patienteneingabe in eine mobile elektronische Vorrichtung erzeugt werden; und
die postoperativen Rückkopplungsdaten von der mobilen elektronischen Vorrichtung empfangen werden.

8. Verfahren nach Anspruch 1, ferner umfassend Bestimmen der Rückkopplung auf der Basis von Empfangen von Bewegungssensordaten von einem Sensor, der einer durch eine Kamera einer mobilen Vorrichtung erfassten Bewegung des Patienten zugeordnet ist.

9. Mindestens ein maschinenlesbares Medium, das Anweisungen zum Betrieb eines Rechensystems einschließt, die, wenn sie durch eine Maschine ausgeführt werden, die Maschine dazu veranlassen, Vorgänge beliebiger der Verfahren nach Anspruch 1-8 durchzuführen.

10. Einrichtung, die Mittel umfasst, die zum Durchführen beliebiger der Verfahren nach Anspruch 1-8 ausgelegt sind.

11. System, umfassend:
einen Prozessor; und
Speicher, der Anweisungen einschließt, die, wenn sie durch den Prozessor ausgeführt werden, den Prozessor dazu veranlassen, Vorgänge zu Folgendem durchzuführen:
nach Abschluss einer orthopädischen Prozedur an Anatomie eines Patienten Bestimmen eines endgültigen Zustands der Anatomie im Anschluss an den Abschluss der orthopädischen Prozedur;
Bestimmen eines postoperativen Genesungsprotokolls für den Patienten auf der Basis des endgültigen Zustands unter Verwendung eines mit maschinellem Lernen trainierten Modells;
wobei das mit maschinellem Lernen trainierte Modell unter Verwendung von postoperativen Genesungsprotokollen, endgültigen Zuständen und postoperativen Rückkopplungsdaten für Patienten trainiert wird, wobei die postoperativen Rückkopplungsdaten auf eine postoperative objektive Ergebnismetrik in Bezug auf das postoperative Patientenbehandlungsprotokoll oder die Anatomie hinweisen; und
eine Anzeigevorrichtung, die dazu konfiguriert ist, eine Benutzerschnittstelle darzustellen, um das postoperative Genesungsprotokoll für den Patienten zu identifizieren.

12. System nach Anspruch 11, wobei die Anweisungen den Prozessor ferner dazu veranlassen, während der orthopädischen Prozedur intraoperative Informationen von einer robotischen chirurgischen Vorrichtung zu empfangen und unter Verwendung der intraoperativen Informationen das postoperative Genesungsprotokoll unter Verwendung des mit maschinellem Lernen trainierten Modells zu erzeugen.

13. System nach Anspruch 11, wobei die Anweisungen den Prozessor ferner zu Folgendem veranlassen:
Empfangen eines vorhergesagten endgültigen Zustands der Anatomie intraoperativ; und
Verwenden des mit maschinellem Lernen trainierten Modells, um ein postoperatives Genesungsprotokoll für den Patienten auf der Basis des vorhergesagten endgültigen Zustands vorherzusagen;
wobei die Anzeigevorrichtung ferner dazu konfiguriert ist, das vorhergesagte postoperative Genesungsprotokoll auf der Benutzerschnittstelle anzuzeigen.

14. System nach Anspruch 11, wobei das mit maschinellem Lernen trainierte Modell ferner unter Verwendung von präoperativen Informationen über den Patienten als Eingabe in das mit maschinellem Lernen trainierte Modell während des Trainings trainiert wird, wobei die präoperativen Informationen auf die postoperative objektive Ergebnismetrik in Bezug auf das postoperative Patientenbehandlungsprotokoll oder die Anatomie hinweisen.

15. System nach einem der Ansprüche 11-14, wobei die postoperativen Rückkopplungsdaten Bewegungsbereichsinformationen einschließen und Schmerzinformationen auf der Basis einer Patienteneingabe in eine mobile elektronische Vorrichtung erzeugt werden; und
die postoperativen Rückkopplungsdaten von der mobilen elektronischen Vorrichtung empfangen werden.

## Revendications

1. Procédé mis en oeuvre par ordinateur comprenant :
la détermination, à l'achèvement d'une procédure orthopédique sur l'anatomie d'un patient, d'un état final de l'anatomie, l'état final de l'anatomie comprenant des informations décrivant l'anatomie du patient suite à l'achèvement de la procédure orthopédique ;
la détermination, à l'aide d'un modèle entraîné par apprentissage automatique, d'un protocole de récupération postopératoire pour le patient sur la base de l'état final, le protocole de traitement postopératoire du patient étant déterminé par le modèle entraîné par apprentissage automatique pour se corréler à des résultats positifs pour des données similaires à celles du patient ;
la réception de données de rétroaction postopératoire provenant d'un dispositif associé au patient, les données de rétroaction postopératoire indiquant une mesure de résultat d'objectif postopératoire relative au protocole de récupération postopératoire ou à l'anatomie ;
la génération d'un protocole de traitement de patient postopératoire modifié sur la base de la mise à jour du modèle entraîné par apprentissage automatique à l'aide de données d'apprentissage sur la base des données de rétroaction postopératoires, du protocole de récupération postopératoire et de l'état final ; et
l'émission d'une recommandation de traitement de patient, la recommandation de traitement de patient étant basée sur le protocole de récupération postopératoire de patient modifié.

2. Procédé selon la revendication 1, comprenant en outre la réception d'informations peropératoires, provenant d'un dispositif chirurgical robotisé, préalablement collectées pendant la procédure orthopédique, et dans lequel la mise à jour du modèle entraîné par apprentissage automatique comprend l'utilisation des informations peropératoires en tant que données d'apprentissage mises à jour.

3. Procédé selon la revendication 1, comprenant en outre :
la réception, de manière peropératoire lors d'une procédure ultérieure, d'un état final prédit de l'anatomie ;
l'utilisation du modèle entraîné par apprentissage automatique pour prédire un protocole de récupération postopératoire pour le patient sur la base de l'état final prédit ; et
la sortie, de manière peropératoire, du protocole de récupération postopératoire prédit pour affichage.

4. Procédé selon la revendication 1, comprenant en outre :
l'enregistrement d'une action entreprise par un dispositif chirurgical robotisé (102) pendant une partie de la procédure ultérieure ;
la détermination d'une recommandation de procédure chirurgicale robotisée, à l'aide du modèle entraîné par apprentissage automatique, la recommandation de procédure chirurgicale robotisée comprenant une modification de la partie de la procédure ultérieure effectuée par le dispositif chirurgical robotisé (102) ; et
la sortie de la recommandation de procédure chirurgicale robotisée en fournissant de manière peropératoire la recommandation de procédure chirurgicale robotisée à un chirurgien utilisant le dispositif chirurgical robotisé (102).

5. Procédé selon la revendication 1, dans lequel l'anatomie est un genou du patient et l'état final comprend un état final du genou sur la base d'au moins un élément parmi une amplitude de mouvement finale, un angle valus/vargus final, une tension ligamentaire finale, une distance d'écartement finale, un type d'implant final et une taille d'implant final.

6. Procédé selon la revendication 1, comprenant en outre la détermination d'une trajectoire de récupération postopératoire du patient sur la base de la rétroaction, du protocole de récupération postopératoire et de l'état final ; et la génération, à l'aide de la rétroaction, du protocole de traitement postopératoire du patient et de l'état final en tant qu'entrées au modèle entraîné par apprentissage automatique, d'une modification du protocole de récupération postopératoire sur la base de la trajectoire de récupération postopératoire du patient.

7. Procédé selon la revendication 1, dans lequel les données de rétroaction postopératoires comprennent des informations sur l'amplitude de mouvement et des informations sur la douleur sont générées sur la base d'une entrée du patient sur un dispositif électronique mobile ; et
les données de rétroaction postopératoire sont reçues du dispositif électronique mobile.

8. Procédé selon la revendication 1, comprenant en outre la détermination de la rétroaction sur la base de la réception de données de capteur de mouvement provenant d'un capteur associé au déplacement du patient capturé par une caméra d'un dispositif mobile.

9. Au moins un support lisible par machine comprenant des instructions pour le fonctionnement d'un système informatique, qui, lorsqu'elles sont exécutées par une machine, amènent la machine à exécuter les opérations selon l'un quelconque des procédés des revendications 1 à 8.

10. Appareil comprenant des moyens adaptés pour exécuter l'un quelconque des procédés des revendications 1 à 8.

11. Système comprenant :
un processeur ; et
une mémoire comprenant des instructions qui, lorsqu'elles sont exécutées par le processeur, amènent le processeur à effectuer des opérations pour :
déterminer, à l'achèvement d'une procédure orthopédique sur l'anatomie d'un patient, un état final de l'anatomie suite à l'achèvement de la procédure orthopédique ;
déterminer, à l'aide d'un modèle entraîné par apprentissage automatique, un protocole de récupération postopératoire pour le patient sur la base de l'état final ;
dans lequel le modèle entraîné par apprentissage automatique est entraîné à l'aide de protocoles de récupération postopératoire, d'états finaux et de données de rétroaction postopératoire pour des patients, les données de rétroaction postopératoire indiquant une mesure de résultat d'objectif postopératoire relative au protocole de traitement postopératoire du patient ou à l'anatomie ; et
un dispositif d'affichage configuré pour présenter une interface utilisateur pour identifier le protocole de récupération postopératoire pour le patient.

12. Système selon la revendication 11, dans lequel les instructions amènent en outre le processeur à recevoir des informations peropératoires, en provenance d'un dispositif chirurgical robotisé, pendant la procédure orthopédique, et à utiliser les informations peropératoires pour générer le protocole de récupération postopératoire à l'aide du modèle entraîné par apprentissage automatique.

13. Système selon la revendication 11, dans lequel lesdites instructions amènent en outre le système à :
recevoir, de manière peropératoire, un état final prédit de l'anatomie ; et
utiliser le modèle entraîné par apprentissage automatique pour prédire un protocole de récupération postopératoire pour le patient sur la base de l'état final prédit ;
dans lequel le dispositif d'affichage est en outre configuré pour afficher le protocole de récupération postopératoire prédit sur l'interface utilisateur.

14. Système selon la revendication 11, dans lequel le modèle entraîné par apprentissage automatique est en outre entraîné en utilisant des informations préopératoires concernant le patient en tant qu'entrée dans le modèle entraîné par apprentissage automatique pendant l'apprentissage, les informations préopératoires indiquant la mesure de résultat d'objectif postopératoire relatif au protocole de traitement postopératoire du patient ou à l'anatomie.

15. Système selon l'une quelconque des revendications 11 à 14, dans lequel les données de rétroaction postopératoires comprennent des informations sur l'amplitude de mouvement et des informations sur la douleur sont générées sur la base d'une entrée du patient sur un dispositif électronique mobile ; et
les données de rétroaction postopératoire sont reçues du dispositif électronique mobile.
